# EUROPEAN PATENT APPLICATION

(11) **EP 3 575 416 A1**
(43) Date of publication of application: **04.12.2019**
(21) Application number: 18175561.2
(22) Date of filing: 01.06.2018
(51) Int. Cl.: C12Q 1/6818, C12N 15/115

(54) **DETECTION OF TARGET MOLECULES BY DETERMINING THE MELTING TEMPERATURE OF A RECOGNITION MODULE**

(71) Applicant: InfanDx AG, 50670 Köln (DE)
(72) Inventor: MAHMOUD, Mostafa Mohamed Safwat Ahmed, 78054 Villingen-Schwenningen (DE); DEIGNER, Hans Peter, 68632 Lampertheim (DE)
(74) Representative: Winter, Brandl, Fürniss, Hübner, Röss, Kaiser, Polte - Partnerschaft mbB

(57) **Abstract**

The present invention relates to a method of detection of at least one target molecule comprising determining the melting temperature change upon binding to at least one recognition molecule, an aptamer and its use in said method, a use of said method for determination of thermodynamic properties of binding between a recognition molecule and a target molecule, a use of said method for the identification of recognition molecules recognizing a known target, and the use of calorimetry for the detection of a target molecule in a liquid sample

SEQUENCE LISTING

<110> InfanDX AG

<120> Detection of target molecules by determining the melting temperature
of a recognition molecule

<130> ID1904P-EP

<160> 5

<170> BiSSAP 1.3.6

<210> 1
<211> 31
<212> DNA
<213> Artificial Sequence

<220>
<223> Aptamer Sequence

<400> 1
attcaatttg aggcgggtgg gtgggttgaa t                                    31

<210> 2
<211> 42
<212> DNA
<213> Artificial Sequence

<220>
<223> Aptamer Sequence

<400> 2
ataccagctt attcaatttg aggcgggtgg gtgggttgaa ta                        42

<210> 3
<211> 21
<212> DNA
<213> Artificial Sequence

<220>
<223> Aptamer Sequence

<400> 3
cacggcatgg tgggcgtcgt g                                               21

<210> 4
<211> 21
<212> DNA
<213> Artificial Sequence

<220>
<223> Scrambled Sequence

<400> 4
attcaatttc tcccgttgaa t                                               21

<210> 5
<211> 32
<212> DNA
<213> Artificial Sequence

<220>
<223> Scrambled Sequence

<400> 5
cacggcatgg ttccatactt aagggcgtcg tg                                    32

## Description

### Technical field

The present invention relates to a method of detection of at least one target molecule comprising determining the melting temperature change upon binding to at least one recognition molecule, an aptamer and its use in said method, a use of said method for determination of thermodynamic properties of binding between a recognition molecule and a target molecule, a use of said method for the identification of recognition molecules recognizing a known target, and the use of calorimetry for the detection of a target molecule in a liquid sample.

### Background of the Invention

The reliable detection and quantification of small molecules, such as metabolites and signalling molecules found endogenously in living organisms or toxins as well as contaminants found in the environment, independent of HPLC and mass spectrometry remains a challenge.

The use of HPLC systems requires expensive equipment and well-trained personnel. On the other hand, biosensors or assays based on antibodies specific for small molecules lack the high throughput and suffer from drawbacks usually associated with the assay format e.g. lack of immunogenicity, the need for labelling in competitive formats or the requirement for two binding epitopes for sandwich formats, as these are hardly compatible with small molecule targets.

In contrast, aptamer-based biosensors can address these drawbacks. They are advantageous over antibodies in terms of cost, stability, reversibility of denaturation, sensitivity, and specificity. Methods based on aptamers for protein detection and quantification vary a lot and offer a variety of approaches such as fluorescence sensors, electrochemical sensors, aptamer arrays and aptamer-gold nanoparticles sensors.

However, due to difference in mass and size, most aptamer-based biosensors for small molecules mentioned in the literature require a complex setup and a sophisticated detection method to achieve the high throughput performance. Moreover, they usually require immobilization of aptamers and/or washing and incubation steps.

Aptamers are RNA/DNA sequences capable of binding to target molecules specifically and with high affinity. They were first introduced in 1990 through an in-vitro selection process known as systemic evolution of ligands by exponential enrichment (SELEX). Various aptamers against small molecules have been reported in the literature (McKeague, M. & DeRosa, M.C. Challenges and Opportunities for Small Molecule Aptamer Development. Journal of Nucleic Acids 2012, 20 (2012).).

The uniqueness of aptamers relates to conformational changes associated with binding. Aptamers adopt different conformational structures based on internal base pairings e.g. stem loops, G-quadruplexes and pseudoknots. These structures are either stabilized or destroyed upon target binding. In fact, this feature of nucleic acids has been used to design molecular beacons to signal the presence of specific target nucleic acid sequence.

Based on this concept, Hamaguchi et al. (Hamaguchi, N., Ellington, A. & Stanton, M. Aptamer beacons for the direct detection of proteins. Anal Biochem 294, 126-131 (2001).) developed an aptamer beacon where target induced conformational change enabled detection and quantification of thrombin using a fluorophore on one end of the loop and a quencher on the other. This homogeneous assay format does not require immobilization of the biorecognition molecules on solid support thus evading the dependence on the relatively slow diffusion kinetics faced by common bioassays, but driving the bioreaction by its fast binding kinetics.

One of the major drawbacks faced by solid phase assays and biosensors lies in the dependence on mass transport rather than the fast assay kinetics. Differential scanning fluorimetry has been used to identify low-molecular-weight ligands that bind and stabilize purified proteins and the governing thermodynamics have been studied extensively. It has been recently shown that quantifying proteins using thermofluorimetric analysis is possible (Hu, J., Kim, J. & Easley, C.J. Quantifying Aptamer-Protein Binding via Thermofluorimetric Analysis. Analytical methods: advancing methods and applications 7, 7358-7362 (2015); Kim, J. et al. Protein quantification using controlled DNA melting transitions in bivalent probe assemblies. Anal Chem 87, 9576-9579 (2015).).

This method, however, depends on a DNA intercalating dye as a reporter molecule e.g. SYBR green. SYBR green has associated drawbacks and limitations which include the shift of melting temperature of the DNA depending on the dye concentration and preferential binding to DNA fragments with higher GC content.

SYBR green is also known to compete with target binding leading to imprecise estimation of the melting temperature. This competition of SYBR green and similar intercalating dyes, has been applied to quantify target binding through fluorescence signal change upon target binding. Moreover, quantification was through the fluorescence signal and peak height and not related to the determination of a melting temperature shift. A similar principle was applied for the quantification of cyclic adenosine monophosphate with an antibody-oligonucleotide construct.

Accordingly, it remains a problem to reliably detect and quantify small molecules, such as metabolites and signalling molecules as well as contaminants found in the environment, independent of the use of HPLC and mass spectrometry.

Therefore, a novel method is required for easy, quick and reproducible determination of the presence of small target molecules and their quantity down to very low concentrations.

It is thus an object of the present invention to provide a novel method for detection of at least one target molecule.

It is a further object of the present invention to provide a novel aptamer molecule which could be used in said method, and its use therein.

It is another object of the present invention to provide a use of this method for determination of thermodynamic binding properties as well as for the identification of recognition molecules able to recognize a known target.

Ultimately, it is another object of the present invention to provide a use of calorimetry for the detection of a target molecule in a liquid sample.

### Summary of the Invention

These objects have been solved by the aspects of the present invention as specified hereinafter.

According to the first aspect of the present invention, a method of detection of at least one target molecule is provided comprising contacting a liquid sample with at least one recognition molecule, wherein the at least one recognition molecule is able to specifically bind to the at least one target molecule, wherein specific binding of the at least one recognition molecule to the at least one target molecule induces a change in the melting temperature of the recognition molecule, and determining the melting temperature of the at least one recognition molecule after contacting with the liquid sample, and comparing the melting temperature of the at least one recognition molecule which has been determined in the step of determining the melting temperature with a standardized curve to identify the presence of the at least one target molecule in the liquid sample.

According to a preferred embodiment of the first aspect of the invention, the melting temperature of the at least one recognition molecule is determined by monitoring the temperature melting curve of the liquid sample.

According to another preferred embodiment of the first aspect of the invention, the detection of the at least one target molecule is a quantitative detection or quantification of the concentration of the at least one target molecule in the sample.

According to another preferred embodiment of the first aspect of the invention, the method for detection of at least one target molecule is based on calorimetry, preferably wherein detection of fluorescence and/or UV absorbance does not form part of the method.

According to yet another preferred embodiment of the first aspect of the invention, a standardized calibration curve for the relation between melting temperatures and concentration of the at least one target molecule is prepared prior to contacting the liquid sample with at least one recognition molecule according to the method of the first aspect of the invention.

According to a preferred embodiment of the first aspect of the invention, the change in the melting temperature of the at least one recognition molecule is detected by measuring a melting profile of the liquid sample, preferably by using a calorimetry device, more preferably by using a quantitative PCR (qPCR) machine.

According to a preferred embodiment of the first aspect of the invention, one recognition molecule specifically binding to one target molecule is present in the liquid sample.

According to another preferred embodiment of the first aspect of the invention, two or more different recognition molecules are contacted with the liquid sample, wherein each of the two or more different recognition molecules is able to specifically bind to a different target molecule and/or wherein each of the two or more recognition molecules has a different temperature melting curve influenced by binding to its respective target molecule.

According to one preferred embodiment of the first aspect of the invention, the recognition molecule(s) is/are selected from the group comprising aptamers, spiegelmers, RNA, DNA, modified nucleic acids, affimers, or the like, preferably wherein the recognition molecule(s) is/are (an) aptamer(s), more preferably wherein the recognition molecule(s) is/are (an) aptamer beacon(s).

According to a preferred embodiment of the first aspect of the invention, the recognition molecule(s) is/are immobilized.

According to another preferred embodiment of the first aspect of the invention, the at least one target molecule is selected from the group comprising small molecules, pharmaceuticals, ions, proteins, peptides, receptors, pollutants, drugs, cells or parts of cells, bacteria, viruses, or the like, preferably wherein the at least one target molecule does not consist of RNA or DNA.

According to yet another preferred embodiment of the first aspect of the invention, a pair of fluorophore and quencher are provided on one or more of the at least one recognition molecules provided in the liquid sample and wherein the pair of fluorophore and quencher provided on one or more of the at least one recognition molecule are brought into spatial proximity upon binding to the target molecule, preferably wherein a pair of fluorophore and quencher are provided on each of the at least one recognition molecules.

According to a preferred embodiment of the first aspect of the invention, a pair of Forster resonance energy transfer (FRET) donor and FRET acceptor molecules are provided on one or more of the at least one recognition molecules provided in the liquid sample and wherein the pair of FRET donor and FRET acceptor molecules provided on one or more of the at least one recognition molecule are brought into spatial proximity upon binding to the target molecule, preferably wherein a pair of FRET donor and FRET acceptor molecules are provided on each of the at least one recognition molecules.

According to one preferred embodiment of the first aspect of the invention, at least one recognition molecule is in the form of an aptamer beacon, and at least one different recognition molecule is not in the form of an aptamer beacon, and these recognition molecules can compete for binding to the target molecule.

According to one more preferred embodiment of the first aspect of the invention, the at least one recognition molecules in the form of an aptamer beacon is labelled, preferably fluorescently labelled, more preferably by a pair of fluorophore and quencher and/or by a pair of FRET donor and FRET acceptor molecules, as defined hereinabove.

According to an even more preferred embodiment of the first aspect of the invention, the at least one recognition molecule in the form of an aptamer beacon has a shorter sequence than the at least one different recognition molecule not in the form of an aptamer beacon.

According to another preferred embodiment of the first aspect of the invention, the method further involves determination of temperature-dependent Circular Dichroism (CD) spectra and comparison with standardized CD spectra and/or defined points of a standardized CD curve.

According to yet another preferred embodiment of the first aspect of the invention, the method further involves determination of UV absorbance and/or a fluorescent signal and comparison with a standardized UV absorbance or fluorescence curve.

According to a more preferred embodiment of the first aspect of the invention, a standardized calibration curve for the relation between the CD spectra and/or the UV absorbance and/or the fluorescent signal and concentration of the target molecule is prepared prior to contacting the liquid sample with at least one recognition molecule according to the method of the first aspect of the invention.

According to an even more preferred embodiment of the first aspect of the invention, the step of comparing the melting temperature of the method of the first aspect of the invention includes comparing the CD spectra and/or the UV absorbance and/or the fluorescent signal determined in the step of determining the melting temperature of the method of the first aspect of the invention with standardized CD spectra or a standardized UV absorbance or fluorescence curve to identify the presence of the at least one target molecule in a sample.

According to the second aspect of the present invention, an aptamer of SEQ ID No. 1 (ATT CAA TTT GAG GCG GGT GGG TGG GTT GAA T ; 31 nucleotide ethanolamine aptamer) is provided.

According to the third aspect of the present invention, a use of the aptamer according to the second aspect of the present invention in the method according to the first aspect of the invention is provided.

According to the fourth aspect of the present invention, a use of the method according to the first aspect of the invention for determination of thermodynamic properties of binding of a recognition molecule to a target molecule is provided.

According to the fifth aspect of the present invention, a use of the method according to the first aspect of the invention for the identification of recognition molecules recognizing a known target is provided.

According to the sixth aspect of the present invention, a use of calorimetry for the detection of a target molecule in a liquid sample by using a recognition molecule able to specifically bind to the target molecule is provided, wherein the recognition molecule bound to the target molecule has a different temperature melting curve in comparison to the recognition molecule alone.

### Description of Figures

Figure 1 shows the melting temperature in °C plotted against the concentration of ampicillin.
Figure 2 shows the melting temperature in °C plotted against the concentration of ethanolamine.
Figure 3 shows the melting temperature of the 42 nt ethanolamine-binding aptamer of SEQ ID No. 2 in °C for the negative control in buffer and upon addition of 1µM phenylethylamine.
Figure 4 shows raw signal data from the qPCR machine for temperature in °C plotted against fluorescence intensity in case of ampicillin and the ampicillin-binding aptamer of SEQ ID No. 3.
Figure 5 shows a first derivative graph of the signal measured over temperature in case of ampicillin and the ampicillin-binding aptamer of SEQ ID No. 3.
Figure 6 shows raw signal data from the qPCR machine for temperature in °C plotted against fluorescence intensity in case of ethanolamine and the 31 nt ethanolamine-binding aptamer of SEQ ID No. 1.
Figure 7 shows a first derivative graph of the signal measured over temperature in case of ethanolamine and the 31 nt ethanolamine-binding aptamer of SEQ ID No. 1.
Figure 8 shows raw signal data from the qPCR machine for temperature in °C plotted against fluorescence intensity in case of ampicillin and the scrampled ampicillin-control sequence of SEQ ID No. 4.
Figure 9 shows a first derivative graph of the signal measured over temperature in case of ampicillin and the scrampled ampicillin-control sequence of SEQ ID No. 4.
Figure 10 shows raw signal data from the qPCR machine for temperature in °C plotted against fluorescence intensity in case of ethanolamine and the scrambled ethanolamine-control sequence of SEQ ID No. 5.
Figure 11 shows a first derivative graph of the signal measured over temperature in case of ethanolamine and the scrambled ethanolamine-control sequence of SEQ ID No. 5.
Figure 12 shows a) the melting profile of different concentrations of the 42 nt ethanolamine-binding aptamer of SEQ ID No. 2 using 10X SYBR green, b) the melting profile of different concentration of the 31 nt ethanolamine-binding aptamer of SEQ ID No. 1 using 10X SYBR green, c) the melting profile of different concentrations of the 42 nt ethanolamine-binding aptamer beacon of SEQ ID No. 2, and d) the melting profile of different concentrations of the 31 nt ethanolamine-binding aptamer beacon of SEQ ID No. 1.

### Detailed Description of the Invention

The present inventors have identified and provide herein a simple method for the detection and quantification of small target molecules based on the shift of the melting temperature of recognition molecules, such as e.g. aptamer beacons. In case of aptamer beacons, the presence of the target molecule leads to either stabilization or destruction of the beacon structure leading to a shift in the melting temperature.

The method can be carried out using any qPCR machine or other machine capable of capturing a melting profile and yields fast results within minutes of analysis time. In comparison to a common FRET experiment, it is simpler, faster and more reproducible. Moreover, the use of the melting profile for the determination of the presence and/or quantity of the target molecule shifts the focus on the melting temperature rather than on a change in fluorescence signal alone.

This minimizes the potential influence of fluorescent background and fluctuations in signal due to non-optimal folding of the recognition molecules such as aptamers or structure switching related with uncontrolled temperatures as opposed to controlled temperature curves inside a device such as a PCR machine. The present invention is considered the first report on using melting temperature shift of recognition molecules such as aptamer beacons to quantify small target molecules.

Applying a temperature gradient significantly accelerates the establishment of partial equilibria, thus reducing assay times. Further, as signals (changes in melting points of e.g. aptamer beacons) are detected along a temperature shift, signals are obtained within any temperature of the gradient and do not require an exactly matching temperature. Since the principle is based on changes of thermodynamics of the whole system it can be much more sensitive than methods applying establishment of static (e.g. strand-displacement based) equilibration.

In addition, a preferred embodiment of the method of the present invention using e.g. a fluorophore-quencher structure in a beacon format is superior to a method applying DNA-intercalating or double-strand DNA-binding dyes such as SYBR-green since thermodynamics in such a system is way too complex to provide clear and separated melting-shift signals by optical readouts.

The inventors' findings pave the way for establishing assays for small molecules in combination with PCR machines or any similar device capable of capturing the melting profile. In general, quantification is performed by measuring a standard curve recording concentration-dependent optical signals across a suitable temperature range to identify melting temperatures at given conditions; measure unknown concentrations of the target by recording the melting temperature(s) alone, and processing the measured melting temperature by an algorithm providing a best fit function for assigning melting temperatures to target molecule concentrations.

An indirect format for target molecule determination is also provided. According to a preferred embodiment of the present invention, this format has an unlabelled binding aptamer and an aptamer beacon competing for the binding region. Through capturing the melting temperature of such a probe, the target molecule could be determined and quantified. Similarly, the method according to the present invention could also give insights on the binding thermodynamics and mechanisms using different probes.

The method of the present invention also has great multiplexing potential. Since aptamers for different targets are of different length, they are expected to have different melting temperatures. In a common binding buffer, different aptamers could bind to their targets at the same time and provide information about their differential melting temperature shifts.

An onsite point of care diagnostic kit is provided making full use of the concept of the method presented here. Based on the advances made in PCR on chip technology, a simple miniaturization of the assay should be possible to achieve and could form the core of said kit.

The challenges and bottlenecks faced by miniaturized PCR assays, e.g. the need for non-metallic heating elements not to interfere with the enzymes for the PCR reaction, would not apply here since the assay is based on profiling the melting temperature and no polymerase reaction is involved. The applications for such a kit would include but not limited to clinical diagnostics, environmental testing, contamination for food and water and testing for drugs of abuse.

The present invention provides a method of detection of at least one target molecule comprising the steps of contacting a liquid sample with at least one recognition molecule, wherein the at least one recognition molecule is able to specifically bind to the at least one target molecule, wherein specific binding of the at least one recognition molecule to the at least one target molecule induces a change in the melting temperature of the recognition molecule, and determining the melting temperature of the at least one recognition molecule after contacting with the liquid sample, and comparing the melting temperature of the at least one recognition molecule which has been determined in the step of determining the melting temperature with a standardized curve to identify the presence of the at least one target molecule in the liquid sample.

According to a preferred embodiment of the present invention, the melting temperature is determined by monitoring the temperature melting curve of the sample to be tested. Such monitoring may be done by any device capable of monitoring and obtaining a temperature melting curve of a sample. Devices which are suitable for said purpose are known to persons skilled in the art and may preferably be selected from PCR machines, preferably from PCR machines capable of performing quantitative PCR (qPCR).

In the present invention, a method of detection may preferably be a method of detecting the presence of at least one target molecule in a sample, more preferably of the presence of a target molecule in a concentration above a given cut-off concentration in a sample. Such cut-off concentration depends on the target molecule, the available corresponding recognition molecules and the cut-off concentration which is specific for the distinct combination of target molecule and recognition molecule. Such cut-off concentration can easily be determined by the skilled person by using routine procedures.

According to another preferred embodiment, a method of detection is a method of quantitative detection or quantification of the concentration of the at least one target molecule in the sample. Quantitative detection or quantification of the concentration may preferably be used interchangeably herein. Quantitative detection of at least one target molecule may preferably mean the determination of at least one target molecule concentration in a sample, more preferably wherein said concentration is at least at or above the cut-off concentration mentioned herein.

Preferably, the term "at least one target molecule" may mean one or more target molecules, more preferably two or more, even more preferably three or more target molecules, further preferably four or more target molecules, further preferably five or more target molecules. According to a particularly preferred embodiment, this term may mean one or two target molecules, more preferably one target molecule. Similarly preferably, the term "at least one recognition molecule" may mean one or more recognition molecules, more preferably two or more, even more preferably three or more recognition molecules. According to a particularly preferred embodiment, this term may mean one or two recognition molecules, more preferably one recognition molecule.

The potential for multiplexing the method of the present invention by providing more than one target molecule and more than one recognition molecule is not particularly limited as long as the recognition molecules used concomitantly have different melting temperatures and/or melting temperature shifts upon binding to their target molecule. According to a preferred embodiment, at least one recognition molecule specifically binding to one target molecule is present in the sample. According to another preferred embodiment, at least one recognition molecule specifically binding to a target molecule is present in the sample for each target molecule present in the sample, more preferably one recognition molecule specifically binding to a target molecule is present in the sample for each target molecule present in the sample. Thus, according to a preferred embodiment, the sample comprises one or more pairs of recognition molecules specifically binding to target molecules.

Specific binding in the context of the present invention may preferably mean any kind of interaction between the target molecule and the recognition molecule which is able to shift the melting temperature or induce a change in the melting temperature of the recognition molecule.

Interestingly, the method of the present invention allows a limit of detection (LOD) of a target molecule which is lower than the K_{D} of the binding of the recognition molecule to the target molecule. For more information on this phenomenon, reference is made to the publication of Heilkenbrinker A. et al., Anal Chem. 2015 Jan 6;87(1):677-85. doi: 10.1021/ac5034819. Epub 2014 Dec 10.

Determination of the melting temperature of the recognition molecule/s alone as well as the melting temperature of the recognition molecule/s after contacting with a sample potentially containing target molecule/s may preferably be determined by using calorimetry. One preferred way of determining melting temperatures is described in the publication "Determination of DNA and RNA Melting Point on UV-VIS Photometer SPECORD® PLUS" from Alexandra Kästner of analytikjena (Lit_UV-01_11_e | 02/2011 | AK).

One prerequisite for the method of the present invention is the availability of a standardized melting temperature curve which correlates melting temperatures of a sample with the concentration in a sample of the target molecule to which the recognition molecule specifically binds. Preferably, such a standardized curve is prepared prior to carrying out the method of the present invention. The preparation of this standardized curve does not require undue experimentation and is well within the skills of an expert in the art. Within the context of the present invention, a standardized curve may be referred to as a calibration curve and *vice versa.*

Preferably, the melting temperature of the at least one recognition molecule is determined by monitoring the temperature melting curve of the liquid sample which is contacted with the recognition molecule, preferably wherein the liquid sample contains the target molecule. More preferably, the change in the melting temperature is detected by measuring a melting profile of the liquid sample, preferably by using a calorimetry device, more preferably by using a device capable of performing quantitative PCR (qPCR).

According to a preferred embodiment of the method of the present invention, the method is not based on detection of fluorescence and/or UV absorbance. According to another preferred embodiment of the method of the present invention, the method does not comprise measurements based on microscale thermophoresis, more preferably the method does not comprise measurements of the mobility of molecules in temperature gradients, even more preferably the method does not comprise measurements of the mobility of molecules, particularly preferably the method is not based on thermophoresis. An overview of this technology is published in Jerabek-Willemsen M et al. Assay and Drug Development Technologies. 2011;9(4):342-353.

According to another preferred embodiment of the present invention, a pair of fluorophore and quencher are provided on one or more of the at least one recognition molecules provided in the liquid sample and wherein the pair of fluorophore and quencher provided on one or more of the at least one recognition molecule are brought into spatial proximity upon binding to the target molecule, preferably wherein a pair of fluorophore and quencher are provided on each of the at least one recognition molecules, more preferably wherein different pairs of fluorophore and quencher are provided on each of the at least one recognition molecules in cases where more than one recognition molecule is used.

According to another preferred embodiment of the present invention, a pair of Forster resonance energy transfer (FRET) donor and FRET acceptor molecules are provided on one or more of the at least one recognition molecules provided in the liquid sample and wherein the pair of FRET donor and FRET acceptor molecules provided on one or more of the at least one recognition molecule are brought into spatial proximity upon binding to the target molecule, preferably wherein a pair of FRET donor and FRET acceptor molecules are provided on each of the at least one recognition molecules, more preferably wherein different pairs of FRET donor and FRET acceptor molecules are provided on each of the at least one recognition molecules in cases where more than one recognition molecule is used.

According to a preferred embodiment, the pairs of fluorophore and quencher or FRET donor and FRET acceptor molecules as mentioned above may be replaced by any pair of molecules which either generate a fluorescent signal when brought in close proximity which is lost again when proximity is no longer given or, alternatively, extinguish a fluorescent signal when brought in close proximity which is regenerated when proximity is lost.

According to a preferred embodiment, the recognition molecule/s is selected from the group comprising aptamers, spiegelmers, RNA, DNA, modified nucleic acids, affimers, or the like, preferably wherein the recognition molecule(s) is/are (an) aptamer(s), more preferably wherein the recognition molecule(s) is/are (an) aptamer beacon(s).

Aptamers as recognition molecules which are able to bind to small target molecules are well-known in the art. Preferred aptamers as recognition molecules can be taken from Pfeiffer F and Mayer G (2016) Selection and Biosensor Application of Aptamers for Small Molecules. Front. Chem. 4:25. doi: 10.3389/fchem.2016.00025, more preferably from Table 1 of this publication.

Preferably, the at least one target molecule is selected from the group comprising small molecules, pharmaceuticals, ions, proteins, peptides, receptors, pollutants, drugs, cells or parts of cells, bacteria, viruses, or the like, more preferably wherein the at least one target molecule does not consist of RNA or DNA. Also, target molecules suitable as target molecules in the method of the present invention may preferably be taken from the aforementioned publication of Pfeiffer F and Mayer G, more preferably from Table 1 thereof.

However, in the present invention, the recognition molecules are not particularly limited as long as they are able to specifically bind to a target molecule and wherein this binding induces a change in the melting temperature of the recognition molecule. Also, the target molecules are not specifically limited as long as they are able to specifically bind to a recognition molecule and wherein this binding induces a change in the melting temperature of the recognition molecule. Preferably, the recognition molecule/s are immobilized.

According to another preferred embodiment, at least one recognition molecule used in the method of the present invention is in the form of an aptamer beacon, and at least one different recognition molecule is not in the form of an aptamer beacon, and wherein these recognition molecules can compete for binding to the target molecule.

Further preferably, the at least one recognition molecules in the form of an aptamer beacon is labelled, preferably fluorescently labelled, more preferably by a pair of fluorophore and quencher and/or by a pair of FRET donor and FRET acceptor molecules, or any other fluorescent label which differs in the generation of a fluorescent signal between close proximity and larger distance as explained above.

More preferably, the at least one recognition molecule in the form of an aptamer beacon has a shorter sequence than the at least one different recognition molecule not in the form of an aptamer beacon.

According to a preferred embodiment, the method of the present invention further involves determination of temperature-dependent Circular Dichroism (CD) spectra and comparison with standardized CD spectra and/or defined points of a standardized CD curve. According to another preferred embodiment, the method of the present invention further involves determination of UV absorbance and/or a fluorescent signal and comparison with a standardized UV absorbance or fluorescence curve.

According to a more preferred embodiment, a calibration curve for the relation between the CD spectra and/or the UV absorbance and/or the fluorescent signal and concentration of the target molecule is prepared prior to carrying out the method of the present invention. According to an even more preferred embodiment, step c) of the method of the invention includes comparing the CD spectra and/or the UV absorbance and/or the fluorescent signal determined in step b) with standardized CD spectra or a standardized UV absorbance or fluorescence curve to identify the presence and/or the concentration of the at least one target molecule in a sample.

According to one preferred embodiment of the present invention, melting curves of aptamer beacons provided with pairs of molecules either generating a fluorescent signal when in proximity or quenching a pre-existing fluorescent signal when brought in proximity may be generated in a PCR machine, more preferably in a PCR machine able to perform qPCR.

According to a particularly preferred embodiment, melting curves are generated in a Roche LightCycler, more preferably in accordance with the determination of fluorescence melting curves as described in Darby, AJ et al. (2002). High throughput measurement of duplex, triplex and quadruplex melting curves using molecular beacons and a LightCycler. Nucleic acids research. 30. e39. 10.1093/nar/30.9.e39.

According to one aspect of the present invention, an aptamer having the sequence of SEQ ID No. 1 is claimed. This aptamer has been found by the inventors to exhibit improved folding and binding to ethanolamine in comparison to sequences disclosed in the prior art. Thus, the present invention also provides the use of the aptamer having the sequence of SEQ ID No. 1 to bind to ethanolamine. According to another aspect of the present invention, the use of the aptamer having the sequence of SEQ ID No. 1 in the method of the present invention is provided.

According to another aspect of the present invention, the method of the present invention is used for determination of thermodynamic properties of binding of a recognition molecule to a target molecule. According to yet another aspect of the present invention, the method of the present invention is used for the identification of recognition molecules recognizing a known target. To this end, target molecules may be provided in a sample and recognition molecules contacted therewith to cause a potential shift in melting temperature curves of the recognition molecule/s. In case that a shift is observed, this can be considered as an indication that the recognition molecule recognizes the target molecule.

According to one aspect of the present invention, the use of calorimetry is provided for the detection of a target molecule in a liquid sample by using a recognition molecule able to specifically bind to the target molecule, wherein the recognition molecule bound to the target molecule has a different temperature melting curve in comparison to the recognition molecule alone.

All embodiments of the present invention as described herein are deemed to be combinable in any combination, unless the skilled person considers such a combination to not make any technical sense.

### Examples

Aptamer beacons (with 5' Fluorescein and 3' DABCYL) were synthesized and purified by Integrated DNA Technologies (Coralville, IA). Ampicillin, ethanolamine, phenylethylamine and SYBR® Green I (10,000x in DMSO) were purchased from Sigma-Aldrich (Darmstadt, Germany).

The following are the sequences of the aptamers used, all of which had 5' Fluorescein and 3' DABCYL and with no labels for use with SYBR green.
31 nt Ethanolamine binding aptamer (SEQ ID No. 1): ATTCAATTTGAGGCGGGTGGGTGGGTTGAAT
42 nt Ethanolamine binding aptamer (SEQ ID No. 2): ATACCAGCTTATTCAATTTGAGGCGGGTGGGTGGGTTGAATA
Ampicillin binding aptamer (SEQ ID No. 3): CACGGCATGGTGGGCGTCGTG
Scrambled sequence used as ampicillin negative (SEQ ID No. 4): ATTCAATTTCTCCCGTTGAAT
Scrambled sequence used as ethanolamine negative (SEQ ID No. 5): CACGGCATGGTTCCATACTTAAGGGCGTCGTG

To account for signal variation and scattering of the data, the on-plate redundancy was at least 4 identical wells per parameter. Additionally, each experiment was repeated at least 4 times to assess both reproducibility and inter-assay variation.

The concentration of the aptamer beacon was fixed at 0.5 µM in all experiments except for the experiments with SYBR green (10x) and the corresponding melting analysis using aptamer beacons at the same concentrations (0.5, 1, 2 and 4 µM).

The beacons were pipetted in a 96 wells PCR plate at a volume of 20 µL in ampicillin binding buffer²⁵ consisting of (20 mM Tris, 100 mM NaCl, 0.02% TWEEN® 20, and 100 mM MgCl₂ at pH 7.6) or ethanolamine binding buffer consisting of (20 mM TRIS, 100 mM NaCl, 5 mM KCl, 2 mM MgCl₂, 1 mM CaCl₂, 0.02% Tween® 20 at pH 7.6).

The analyte concentration in case of ampicillin was varied from (0.5 to 32 pM) and were added in a volume of 2 µL per well. In case of ethanolamine and phenylethylamine, the analyte was varied from (5 to 100 nM).

The PCR machine (LightCycler® 480, Roche) was set to heat first till 99 °C followed by holding for 5 minutes to ensure the complete denaturing of any DNA base pairings. Then, the fluorescence was measured from 95 °C to 20 °C with the SYBR Green filter (465 nm excitation and 510 nm emission). As for the ampicillin, we found that heating to 99 °C can possibly lead to its degradation. Therefore, the PCR was set to heat only to 70 °C when detecting ampicillin.

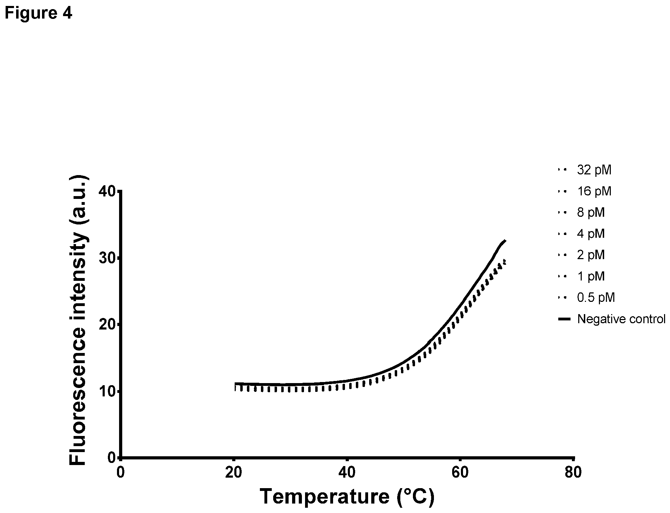

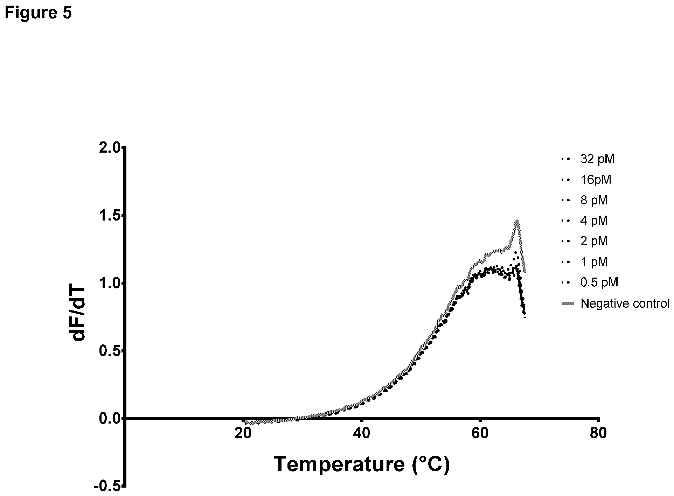

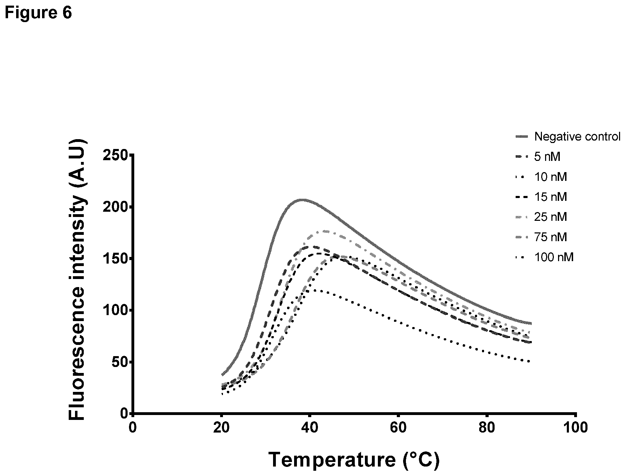

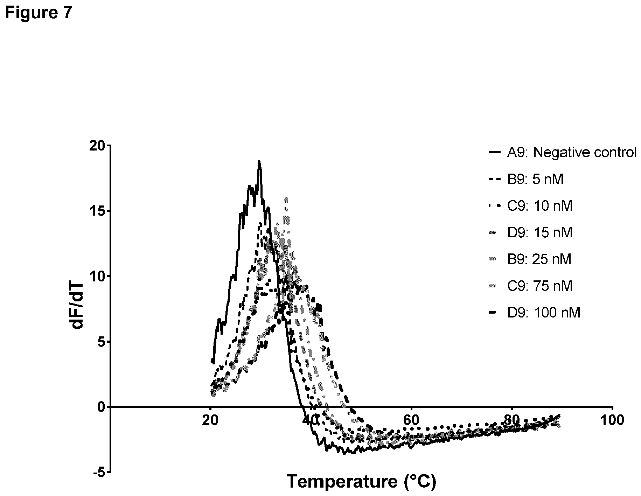

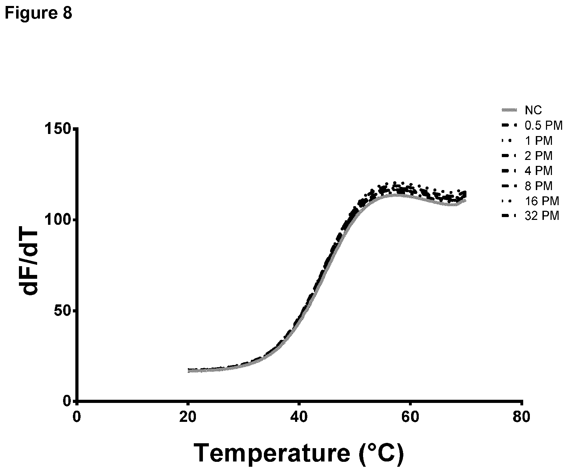

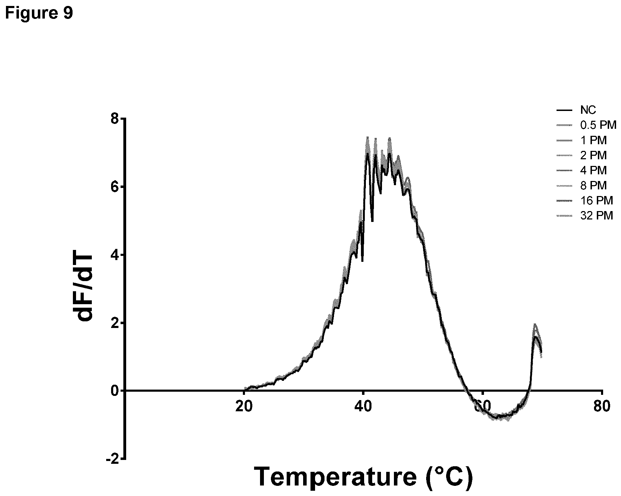

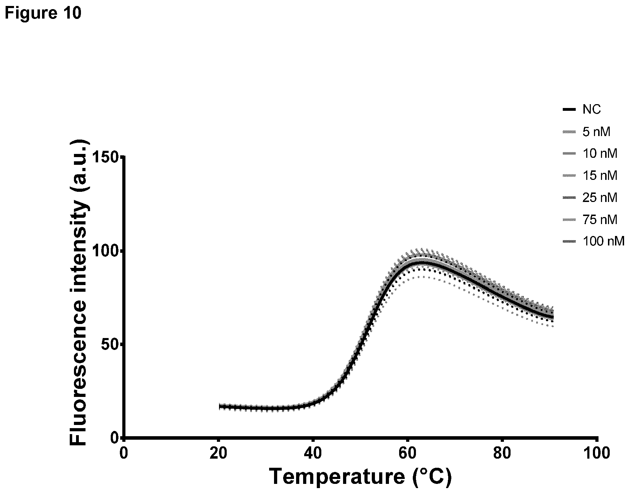

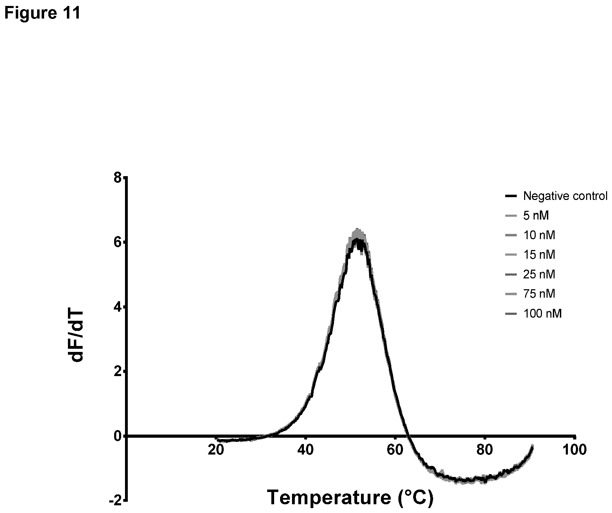

Data analysis (plotting and fitting using one site binding equation) was performed using GraphPad Prism version 7.00 for Windows, GraphPad Software, La Jolla California USA, www.graphpad.com and JMP®, Version <*13.1*>, First Derivative with 2^{nd} order smoothing (4 neighbors). SAS Institute Inc., Cary, NC, 1989-2007. The raw data obtained from the PCR (Figures 4, 6, 8 and 10) was used to generate first derivative graphs for each individual experiment (Figures 5, 7, 9 and 11).

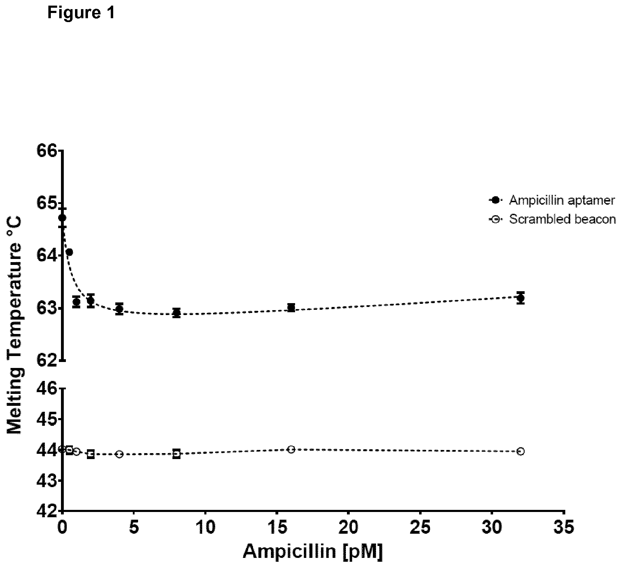

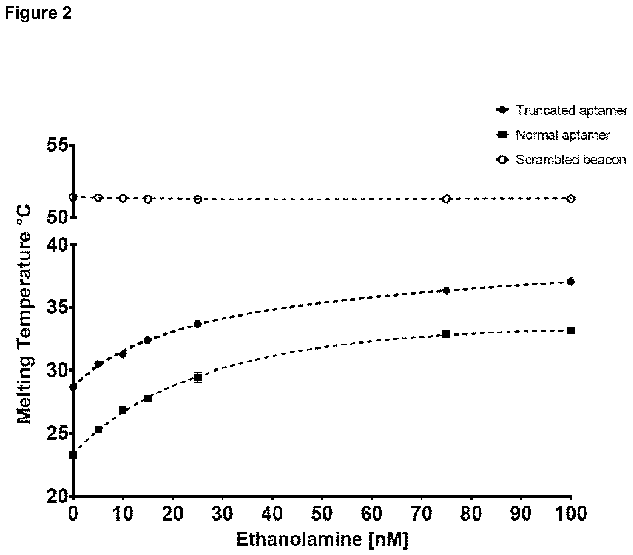

These first derivatives were fitted using a Gaussian distribution with the following equation Y=Amplitude*exp(-0.5*((X-Mean)/SD)^2. The resulting Tm (defined as the Mean in the equation) was then plotted against the concentration (Fig. 1 and 2) to produce the standardized curves/calibration curves.

### Example 1:

As proof of concept and feasibility of the approach presented herein, the ampicillin aptamer beacon (AMP4) of SEQ ID No. 3 was chosen. The secondary structure was predicted using mfold http://www.bioinfo.rpi.edu/applications/mfold to show a conserved region in the loop and a base pairing in the stem which is thought to play an important role in the binding (Song, K.-M., Jeong, E., Jeon, W., Cho, M. & Ban, C. Aptasensor for ampicillin using gold nanoparticle based dual fluorescence-colorimetric methods. Analytical and Bioanalytical Chemistry 402, 2153-2161 (2012).).

Therefore, the presence of the target in the sample will lead to a destabilization of the stem loop structure in case of the ampicillin-binding aptamer beacon decreasing the energy needed to break the stem loop structure and hence decrease the melting temperature.

PCR plates containing the aptamer solution at 0.5 µM concentration per well and a volume of 20 µL were prepared. Then, 2 µL of ampicillin solution (0.5 pM to 32 pM) were pipetted in the wells in such a manner that there are 12 replicates per plate. The plates were then transferred to a lightcycler 480 for acquisition of the melting profile.

As shown in Figure 1, the presence of ampicillin decreased the melting temperature in comparison to the negative control (only buffer with no ampicillin). The method allowed the detection of concentrations as low as 1.5 ± 0.38 pM with an estimated K_{d} value of 0.621 ± 0.33 pM. The limit of detection was defined as (blank mean value + 3*SD_{blank}). As we expected the aptamer-ampicillin binding event leads to destabilization of the stem loop structure and thus to decrease in the energy needed to melt the aptamer beacon.

In case of ampicillin, it is well documented that the compound is highly unstable in high temperatures which leads to its hydrolysis (Mitchell, S.M., Ullman, J.L., Teel, A.L. & Watts, R.J. pH and temperature effects on the hydrolysis of three β-lactam antibiotics: ampicillin, cefalotin and cefoxitin. Sci Total Environ 466-467, 547-555 (2014).; Hou, J.P. & Poole, J.W. Kinetics and Mechanism of Degradation of Ampicillin in Solution. Journal of Pharmaceutical Sciences 58, 447-454 (1969).).

The hydrolysis reaction follows a pseudo first-order kinetics with respect to the initial concentration of ampicillin. This leads to the observed fast saturation within the calibration curve. This is, therefore, not due to the saturation of the aptamer beacons but rather due to the exponential degradation of the substrate. The counterproductive effects of the ampicillin degradation, however, restrict the quantification using thermofluorimetric analysis. Nevertheless, we could still detect amounts as low as 1.5 pM through setting the maximum temperature to 70°C.

### Example 2:

As another example, ethanolamine-binding aptamers were used to further demonstrate the generalizability of the approach and show the case of stabilizing the folded beacon structure. It was previously shown that the ethanolamine aptamer G rich consensus region adopts a G-quartet structure and that this sequence is responsible for the binding.

Thus, we chose a 42 nucleotides sequence ethanolamine-binding aptamer (SEQ ID No. 2; labelled as "normal aptamer" in Figure 2) which is a truncated version of the original full-length 96 nucleotides aptamer (Mann, D., Reinemann, C., Stoltenburg, R. & Strehlitz, B. In vitro selection of DNA aptamers binding ethanolamine. Biochemical and biophysical research communications 338, 1928-1934 (2005).).

This aptamer was further modified by adding a fluorophore (fluorescein) on the 5' end and a quencher (DABCYL) on the 3' end. The folding structure was predicted using mfold and did not show a perfect stem loop folding. Therefore, we decided to modify the sequence by truncating it further to 31 nucleotides (SEQ ID No. 1; labelled as "truncated aptamer" in Figure 2) to improve the folding. Moreover, this allowed us to check the effect of small modifications on the binding parameters.

Our results showed that even a modified structure was still capable of binding to the target. Further, the structural conformation only needs to provide a proximity between the fluorophore and the quencher to work and this may be provided by other means than stem loop e.g. by short complementary strands.

As seen in Figure 2, the addition of ethanolamine shifted the melting temperature through stabilizing the folded beacon structure. The limit of detection (LOD= blank mean value + 3*SD_{blank}) for the 42nt aptamer version was 2.2 ± 0.378 nM and the 31nt truncated version was 1.5 ± 0.615 nM. The estimated Kd value was 26.6 ± 4.4 nM and 28.8 ± 3.4 respectively.

We could show that even small modifications in the structure of the aptamer do not affect the binding (provided that the binding sequence is still present). The approach shows promising results that could be potentially transferred to other aptamers for small molecules.

### Example 3:

The response of the assay using the 42 nt ethanolamine-binding aptamer of SEQ ID No. 2 to the structurally similar molecule phenylethylamine has further been studied.

It has previously been shown that phenylethylamine binds the full-length 96nt aptamer (see above) by affinity elution test at about 50% of the ethanolamine binding (Reinemann, C., Stoltenburg, R. & Strehlitz, B. Investigations on the Specificity of DNA Aptamers Binding to Ethanolamine. Analytical chemistry 81, 3973-3978 (2009).).

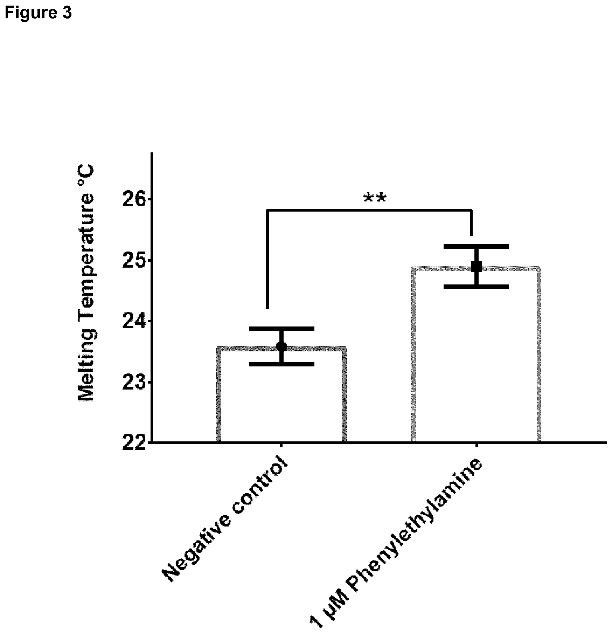

Our results (Fig. 3), indicate, that using an increased concentration of 1 µM phenylethylamine, the 42 nucleotides aptamer of SEQ ID No. 2 showed a significant change in comparison to the control (Figure 3). However, other aptamers with a specificity towards phenylethylamine are likely to successfully detect and quantify much lower concentrations of phenylethylamine.

### Example 4:

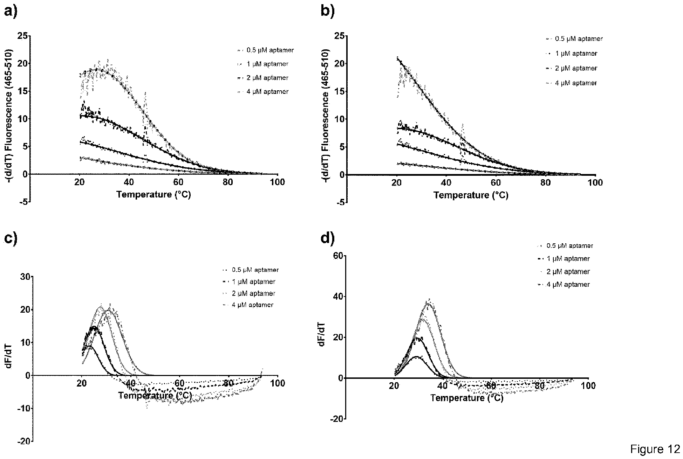

In this example, SYBR green was tested as a candidate intercalating dye (Figure 12 a and b), however, the melting profile could not be captured for the aptamer concentrations used (0.5, 1, 2 and 4 µM and 10X SYBR green). The beacons, however, showed well-defined melting peaks (Figure 12c and d).

## Claims

1. Method of detection of at least one target molecule comprising:
a) Contacting a liquid sample with at least one recognition molecule, wherein the at least one recognition molecule is able to specifically bind to the at least one target molecule, wherein specific binding of the at least one recognition molecule to the at least one target molecule induces a change in the melting temperature of the recognition molecule, and
b) Determining the melting temperature of the at least one recognition molecule after contacting with the liquid sample, and
c) Comparing the melting temperature of the at least one recognition molecule which has been determined in step b) with a standardized calibration curve to identify the presence of the at least one target molecule in the liquid sample.

2. Method of claim 1, wherein the melting temperature of the at least one recognition molecule is determined by monitoring the temperature melting curve of the liquid sample, more preferably wherein the detection of the at least one target molecule is a quantitative detection or quantification of the concentration of the at least one target molecule in the sample.

3. Method of claim 1 or 2, wherein the method for detection of at least one target molecule is based on calorimetry, preferably wherein detection of fluorescence and/or UV absorbance does not form part of the method.

4. Method of any of claims 1 to 3, wherein the change in the melting temperature of the at least one recognition molecule is detected by measuring a melting profile of the liquid sample, preferably by using a calorimetry device, more preferably by using a quantitative PCR (qPCR) machine.

5. Method of any of claims 1 to 4, wherein the recognition molecule(s) is/are selected from the group comprising aptamers, spiegelmers, RNA, DNA, modified nucleic acids, affimers, or the like, preferably wherein the recognition molecule(s) is/are (an) aptamer(s), more preferably wherein the recognition molecule(s) is/are (an) aptamer beacon(s), even more preferably wherein the recognition molecule(s) is/are immobilized.

6. Method of any of claims 1 to 5, wherein the at least one target molecule is selected from the group comprising small molecules, pharmaceuticals, ions, proteins, peptides, receptors, pollutants, drugs, cells or parts of cells, bacteria, viruses, or the like, preferably wherein the at least one target molecule does not consist of RNA or DNA.

7. Method of any of claims 1 to 6, wherein a pair of fluorophore and quencher are provided on one or more of the at least one recognition molecules provided in the liquid sample and wherein the pair of fluorophore and quencher provided on one or more of the at least one recognition molecule are brought into spatial proximity upon binding to the target molecule, preferably wherein a pair of fluorophore and quencher are provided on each of the at least one recognition molecules, alternatively wherein a pair of Forster resonance energy transfer (FRET) donor and FRET acceptor molecules are provided on one or more of the at least one recognition molecules provided in the liquid sample and wherein the pair of FRET donor and FRET acceptor molecules provided on one or more of the at least one recognition molecule are brought into spatial proximity upon binding to the target molecule, preferably wherein a pair of FRET donor and FRET acceptor molecules are provided on each of the at least one recognition molecules.

8. Method of any of claims 1 to 7, wherein at least one recognition molecule is in the form of an aptamer beacon, and wherein at least one different recognition molecule is not in the form of an aptamer beacon, and wherein these recognition molecules can compete for binding to the target molecule, preferably wherein the at least one recognition molecules in the form of an aptamer beacon is labelled, more preferably fluorescently labelled, even more preferably by a pair of fluorophore and quencher and/or by a pair of FRET donor and FRET acceptor molecules, as defined in claim 7.

9. Method of claim 8, wherein the at least one recognition molecule in the form of an aptamer beacon has a shorter sequence than the at least one different recognition molecule not in the form of an aptamer beacon.

10. Method of any of claims 1 to 9, wherein the method further involves determination of temperature-dependent Circular Dichroism (CD) spectra and comparison with standardized CD spectra and/or defined points of a standardized CD curve, alternatively wherein the method further involves determination of UV absorbance and/or a fluorescent signal and comparison with a standardized UV absorbance or fluorescence curve.

11. Method of claim 10, wherein a calibration curve for the relation between the CD spectra and/or the UV absorbance and/or the fluorescent signal and concentration of the target molecule is prepared prior to step a), preferably wherein step c) includes comparing the CD spectra and/or the UV absorbance and/or the fluorescent signal determined in step b) with standardized CD spectra or a standardized UV absorbance or fluorescence curve to identify the presence of the at least one target molecule in a sample.

12. Aptamer having the sequence of SEQ ID No. 1.

13. Use of the aptamer of claim 12 in the method of any of claims 1 to 11.

14. Use of the method of any of claims 1 to 11 for determination of thermodynamic properties of binding of a recognition molecule to a target molecule.

15. Use of the method of any of claims 1 to 11 for the identification of recognition molecules recognizing a known target.

16. Use of calorimetry for the detection of a target molecule in a liquid sample by using a recognition molecule able to specifically bind to the target molecule, wherein the recognition molecule bound to the target molecule has a different temperature melting curve in comparison to the recognition molecule alone.
